(19) Europäisches Patentamt
European Patent Office
Office européen des brevets

(11) **EP 2 910 966 A1**

(12) **EUROPÄISCHE PATENTANMELDUNG**

(43) Veröffentlichungstag:
26.08.2015 Patentblatt 2015/35

(51) Int Cl.:
**G01R 33/563** *(2006.01)*  *G01R 33/483* *(2006.01)*
**G01R 33/565** *(2006.01)*

(21) Anmeldenummer: **15154066.3**

(22) Anmeldetag: **06.02.2015**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **20.02.2014 DE 102014203113**

(71) Anmelder: **Siemens Aktiengesellschaft 80333 München (DE)**

(72) Erfinder: **Stemmer, Alto 91054 Erlangen (DE)**

(54) **Mehrschicht-MRT mit bewegtem Tisch**

(57) Es wird ein Verfahren zur Erzeugung von Bilddaten (BD) eines Untersuchungsobjekts (O) mittels eines Magnetresonanztomographen (3) beschrieben, bei dem während einer Magnetresonanzmessung das Untersuchungsobjekt (O) relativ zu einem Magneten-/Gradienten-Systems (5) des Magnetresonanztomographen (3) mehrfach zwischen einer Anfangsposition (PS) und einer Endposition (PE) hin- und hergefahren wird und dabei jeweils Teile (BRT) eines Bildgebungs-Rohdatensatzes (BR), welcher zur Rekonstruktion von Bilddaten (BD) für Schichten (SL) eines Bildstapels (ST) benötigt wird, in unterschiedlichen Fahrten von der Anfangsposition (PS) zur Endposition (PE) und/oder der Endposition (PE) zur Anfangsposition (PS) akquiriert werden. Darüber hinaus wird ein Magnetresonanztomographiesystem (1) beschrieben, an dem mit einem solchen Verfahren Bilddaten (BD) erzeugt werden können.

FIG 1

**Beschreibung**

Die Erfindung betrifft ein Verfahren zur Erzeugung von Magnetresonanzbilddaten eines Untersuchungsobjekts mittels eines Magnetresonanztomographen, bei dem während einer Magnetresonanzmessung das Untersuchungsobjekt z. B. auf einem Gerätetisch bzw. einer Patientenliege relativ zu einem Magneten-/Gradienten-System des Magnetresonanztomographen verfahren wird und dabei Bildgebungs-Rohdaten akquiriert werden. Basierend auf diesen Bildgebungs-Rohdaten können dann Bilddaten der Schichten rekonstruiert werden. Darüber hinaus betrifft die Erfindung ein Magnetresonanztomographiesystem mit einem Magnetresonanztomographen sowie einer Steuervorrichtung, um ein solches Verfahren durchzuführen.

In einem Magnetresonanzsystem wird üblicherweise der zu untersuchende Körper mit Hilfe eines Grundfeldmagnetsystems einem relativ hohen Grundmagnetfeld, dem sogenannten "B0-Feld", beispielsweise von 3 oder 7 Tesla, ausgesetzt. Zusätzlich wird mit Hilfe eines Gradientensystems ein Magnetfeldgradient angelegt. Über ein Hochfrequenz-Sendesystem werden dann mittels geeigneter Antenneneinrichtungen hochfrequente Anregungssignale (HF-Pulse), das sogenannten "B1-Feld", ausgesendet, was dazu führen soll, dass die Kernspins bestimmter, durch dieses Hochfrequenzfeld resonant angeregter Atome ortsaufgelöst um einen definierten Flipwinkel gegenüber den Magnetfeldlinien des Grundmagnetfelds verkippt werden. Bei der Relaxation der Kernspins werden wiederum Hochfrequenzsignale, sogenannte Magnetresonanzsignale, abgestrahlt, die mittels geeigneter Empfangsantennen empfangen und dann weiter verarbeitet werden. Dabei erfolgt die Datenaufnahme z.B. zeilenweise im Ortsfrequenzraum, dem sogenannten "k-Raum". Auf Basis dieser Rohdaten erfolgt dann unter Anwendung einer Fourier-Transformation eine Rekonstruktion der Bilddaten, welche ein Abbild vom Inneren des Untersuchungsobjekts im "realen" Ortsraum darstellen.

Frühe MR-Systeme benutzen dieselbe Spule als Sende- und Empfangsspule, nämlich eine fest im Tomographen eingebaute sogenannten "Volumenspule" oder "Bodycoil". Ein typischer Aufbau einer Volumenspule ist eine Käfigantenne (Birdcage-Antenne), welche aus mehreren Sendestäben besteht, die parallel zur Längsachse verlaufend um einen Patientenraum des Tomographen herum angeordnet sind, in dem sich ein Patient bei der Untersuchung befindet. Stirnseitig sind die Antennenstäbe jeweils ringförmig kapazitiv miteinander verbunden. Heutzutage wird die Volumenspule oft nur als Sendespule während der Hochfrequenzeinstrahlung eingesetzt, um ein möglichst homogenes B1-Feld senkrecht zur Richtung des Grundmagnetfeldes zu erzeugen. Der Signalempfang erfolgt dagegen meist mit mehreren dedizierten Empfangsspulen, üblicherweise als "Lokalspulen" bezeichnet, die möglichst nahe am zu untersuchenden Organ des Patienten platziert sind.

Messungen mit kontinuierlich durch den Magneten des Magnetresonanztomographen fahrendem Tisch dienen dazu, das Gesichtsfeld (engl.: "field of view") in Richtung der Tischverschiebung (FOVz) zu erweitern und gleichzeitig den Messbereich innerhalb des Magneten z. B. auf einen kleinen Bereich um das Isozentrum des Magnetresonanztomographen, also den Ort höchster Homogenität des Magnetfeldes und größter Linearität des Gradienten-Systems, zu beschränken. Eine mit dem kontinuierlichen Tischvorschub konkurrierende Technik ist die Aufnahme des in Tischvorschubrichtung erweiterten FOV in mehreren Stationen bei jeweils stehendem Tisch. Dabei wird, nachdem alle Daten einer Station akquiriert sind, der Patient mit der Patientenliege zur nächsten Station gefahren und die Messung während der Fahrt ausgesetzt.

Klassisch kommen bei Akquisitionstechniken mit kontinuierlichem Vorschub der Patientenliege vornehmlich Sequenzen mit sehr kurzer Repetitionszeit (üblicherweise und im Folgenden als TR bezeichnet) zum Einsatz.

Hierzu gehören beispielsweise Sequenzen wie TrueFISP (engl. "True fast imaging with steady state precession") oder Protonendichte-gewichtete FLASH- (engl. "Fast Low Angle Shot") Sequenzen. Bei Sequenzen mit sehr kurzer Repetitionszeit ist es möglich, fortlaufend (sukzessiv) die Bildgebungs-Rohdaten einer einzigen Schicht im Zentrum des Magneten zu akquirieren, während der Patient (oder allgemein das Untersuchungsobjekt) mit konstanter Geschwindigkeit

$$V_{table} = \frac{d}{N_{exc}TR} \qquad (1)$$

durch die Anlage gefahren wird. Dabei bezeichnet TR die Zeit zwischen der sukzessiven Anregung einer Schicht und $N_{exc}$ die Zahl der Anregungen pro Schicht, die notwendig ist, um die Bildgebungs-Rohdaten zur Kodierung eines Bildes zu akquirieren. Bei Verwendung einer kartesischen Akquisitionstechnik ist $N_{exc}$ im einfachsten Fall (ohne Einsatz von parallelen Akquisitionstechniken) beispielsweise gleich der Zahl der Phasenkodierschritte pro Schicht. Bei Verwendung einer radialen Akquisitionstechnik ist $N_{exc}$ im einfachsten Fall (eine Speiche pro Anregung) gleich der Zahl der gemessenen Speichen pro Bild. In der Formel (1) ist d den Abstand zwischen benachbarten Schichten (gemessen von Mitte zu Mitte).

Bei dieser sukzessiven Akquisitionstechnik werden die Daten einer ersten Schicht komplett akquiriert, bevor mit der Datenakquisition einer weiteren Schicht begonnen wird.

Darüber hinaus wird inzwischen eine Technik mit kontinuierlichem Tischvorschub angeboten, bei der auch Sequenzen mit moderaten TR (wie z. B. bei T1-gewichteter Bildgebung mit FLASH) bzw. langem TR (wie z. B. bei T2-gewichteten Turbo-Spin-Echosequenzen) zum Einsatz kommen. Da die klassische Aufnahmetechnik mit kontinuierlichem Tischvorschub (also die sukzessive Aufnahme jeweils der Schicht, die gerade das Isozentrum der Anlage durchfährt) hierbei infolge der langen Repetitionszeit TR zu einer extrem langsamen Tischgeschwindigkeit und damit zu einer extrem langen Untersuchungsdauer (und entsprechend niedriger Effizienz) führen würde, setzt man in der Regel eine Verschachtelungstechnik ein, bei der die Daten jeder Schicht an unterschiedlichen Positionen innerhalb des Magnetresonanztomographen akquiriert werden. Diese Akquisitionstechnik steigert die Effizienz gegenüber der klassischen Akquisitionstechnik, gibt aber auch den eigentlichen Vorteil der Techniken mit kontinuierlichem Tischvorschub auf, nämlich die Aufnahme aller Schichten und Daten nahe am Isozentrum. Vielmehr führt die Verschachtelung dazu, dass unterschiedliche Daten ein- und derselben Schicht (im Untersuchungsobjekt bzw. Patienten) notwendigerweise an verschiedenen Orten innerhalb des Magnetresonanztomographen aufgenommen werden. Dies ist prinzipiell eine neue mögliche Artefakt-Quelle, da an verschieden Orten innerhalb des Magnetresonanztomographen wegen der nicht perfekten Homogenität des Magnetfeldes und der nicht perfekten Linearität des Gradienten-Systems unterschiedliche Aufnahmebedingen bestehen.

Es ist eine Aufgabe der vorliegenden Erfindung, ein Verfahren sowie ein geeignetes Magnetresonanztomographiesystem zu schaffen, bei denen Sequenzen mit langem TR effizient mit kontinuierlichem Tischvorschub genutzt werden können, aber das Risiko von Artefakten aufgrund von Feldinhomogenitäten zwischen verschiedenen Messpositionen verringert wird.

Diese Aufgabe wird zum einen durch ein Verfahren nach Patentanspruch 1 und zum anderen durch ein Magnetresonanztomographiesystem nach Patentanspruch 10 gelöst.

Bei dem erfindungsgemäßen Verfahren werden, wie eingangs beschrieben, während einer Magnetresonanzmessung bei Bewegung des Untersuchungsobjekts relativ zum Magneten-/Gradienten-System des Magnetresonanztomographen, z. B. gleichzeitigem Tischvorschub, Bildgebungs-Rohdaten des Untersuchungsobjekts erfasst. Die Bewegung kann dabei kontinuierlich mit einer gleichmäßigen Geschwindigkeit, aber auch mit variierender Geschwindigkeit erfolgen, wobei es ebenso möglich ist, dass die Bewegung zwischenzeitlich angehalten und dann nach einer bestimmten Pause weitergefahren wird.

Dabei werden Bildgebungs-Rohdaten eines Bildstapels bevorzugt im Rahmen einer 2D-axialen Messung erfasst, wobei jeweils, vorzugsweise relativ dünne, 2D-Schichten aufgenommen werden und dabei durch einen davon gebildeten Schichtstapel ein bestimmtes zweidimensionales Volumen dicht erfasst wird. Dabei erfolgt die Schichtselektion in der in der Bewegungsrichtung (z. B. Tischvorschubrichtung) verlaufenden z-Richtung durch ein geeignetes gleichzeitiges Schalten von Gradientenpulsen und Hochfrequenzpuls. Die Ortsauflösung in x- und y-Richtung, senkrecht zur z-Richtung, erfolgt mit Hilfe des Auslesegradienten, welcher meist von rechts nach links bezüglich des Patienten geschaltet wird, und eines Phasencodierungsgradienten, welcher dann senkrecht zum Tisch in Anterior-/Posterior-Richtung bezüglich des Patienten verläuft. Im Rahmen der vorliegenden Erfindung erfolgt die Auslesung der Bildgebungs-Rohdaten bevorzugt senkrecht zu einer Tischvorschubrichtung bzw. die Bewegungsrichtung verläuft senkrecht zur Bildebene.

Erfindungsgemäß wird nun während einer Magnetresonanzmessung das Untersuchungsobjekt relativ zum Magneten-/Gradienten-System mehrfach zwischen einer Anfangsposition und einer Endposition hin- und hergefahren (d. h. wie üblich wird der Patient auf dem beweglichen Tisch im Magneten-/Gradienten-System verfahren und/oder das Magneten-/Gradienten-System wird verfahren) und dabei werden jeweils Teile eines Bildgebungs-Rohdatensatzes, welcher zur Rekonstruktion von Bilddaten für Schichten eines zuvor festgelegten Bildstapels benötigt wird, in unterschiedlichen Fahrten von der Anfangsposition zur Endposition und/oder der Endposition zur Anfangsposition akquiriert. Mit anderen Worten, die Akquisition der Bildgebungs-Rohdaten erfolgt so, dass die Bildgebungs-Rohdaten eines bestimmten, d. h. ein- und desselben Bildstapels, aus mehreren Fahrten stammen.

Auf Basis dieser Bildgebungs-Rohdaten können dann in üblicher Weise Bilddaten der Schichten rekonstruiert werden. Unter Bildgebungs-Rohdaten sind dabei die Rohdaten im k-Raum, zu verstehen, aus denen die Bilddaten rekonstruiert werden, das heißt nicht z. B. irgendwelche Justage- oder Kalibrierungsdaten bzw. Spulensensitivitäten, die ggf. gleichzeitig während der Fahrten oder (wie meist bisher) in weiteren separaten Fahrten erfasst werden können.

Vorzugsweise handelt es sich dabei um Bildgebungs-Rohdaten für Bilddaten eines einzelnen Kontrasts (d.h. beispielsweise protonendichte-gewichteter Kontrast, T1-gewichteter Kontrast, T2-gewichteter Kontrast, diffusionsgewichteter Kontrast) des Bildstapels, die aus mehreren Fahrten stammen. Dies gilt insbesondere auch dann, wenn für die Schichten eines Bildstapels insgesamt Bilddaten mit unterschiedlichen Kontrasten aufgenommen werden.

Die vorliegende Erfindung führt also eine neue Aufnahmetechnik ein, bei der die Daten einer Schicht, wie bei der klassischen Technik mit kontinuierlichem Tischvorschub, ausschließlich dann akquiriert werden können, wenn die Schicht das Isozentrum oder einen engen Bereich um das Isozentrum des Magnetresonanztomographen durchfährt. Unterschiedliche Anregungen einer bestimmten Schicht und/oder benachbarter Schichten eines Schichtstapels können dabei, wie die späteren Beispiele zeigen, vorteilhaft in verschiedenen, direkt oder indirekt aufeinanderfolgenden Fahrten erfolgen. Durch diese "Shuttle-Technik" werden die Vorteile der klassischen Technik beibehalten, die Tischgeschwindigkeit und damit die Effizienz werden aber von den kontrastbestimmenden Parametern (insbesondere der Repetitionszeit TR) entkoppelt. Die erfindungsgemäße Technik ist daher besonders geeignet für Sequenzen, bei denen es zwischen den verschiedenen Anregungen einer bestimmten Schicht bevorzugt zu einer vollständigen Relaxation des Gewebes kommt, also insbesondere T2-gewichtete Bildgebungen mit Turbo-Spin-Echo-Sequenzen und diffusionsgewichtete Bildgebung mit Spin-Echo-Echoplanar-Sequenzen (EPI). Jedoch ist die Erfindung nicht auf solche Sequenzen beschränkt.

Ein erfindungsgemäßes Magnetresonanztomographiesystem benötigt, wie oben erwähnt, neben dem Magnetresonanztomographen mit einem Magneten-/Gradienten-System eine entsprechende Steuervorrichtung, welche ausgebildet ist, um den Magnetresonanztomographen bei einer Magnetresonanzmessung so anzusteuern, dass das Untersuchungsobjekt relativ zu einem Magneten-/Gradienten-System des Magnetresonanztomographen mehrfach zwischen einer Anfangsposition und einer Endposition hin- und hergefahren wird und dabei jeweils Teile eines Bildgebungs-Rohdatensatzes, welcher zur Rekonstruktion von Bilddaten für Schichten eines Bildstapels benötigt wird, in unterschiedlichen Fahrten zwischen der Anfangsposition und der Endposition (d. h. von der Anfangsposition zur Endposition und/oder der Endposition zur Anfangsposition) akquiriert werden.

Ein solches Magnetresonanztomographiesystem weist bevorzugt auch eine geeignete Rekonstruktionseinheit auf, welche ausgebildet ist, um auf Basis der in den verschiedenen Fahrten akquirierten Bildgebungs-Rohdaten Bilddaten der Schichten des Bildstapels zu rekonstruieren.

Beispielsweise könnte die Rekonstruktionseinrichtung hierzu eine Rohdatensortier- und/oder Puffereinheit aufweisen, in der die Bildgebungs-Rohdaten aus den verschiedenen Fahrten jeweils passend zu den Schichten sortiert zwischengespeichert werden, bis die erforderlichen Bildgebungs-Rohdaten zur Rekonstruktion der Bilddaten für die betreffende Schicht vorliegen. Erst dann erfolgt in einer (an sich herkömmlichen) Rekonstruktionseinheit der Rekonstruktionseinrichtung die Bildrekonstruktion.

Diese Rekonstruktionseinrichtung kann auch Teil der Steuervorrichtung des Magnetresonanztomographiesystems sein. Sie kann aber auch auf einem separaten Rechner des Magnetresonanztomographiesystems realisiert sein, beispielsweise einem daran angeschlossenen Terminal etc. Insbesondere können die Steuervorrichtung und/oder die Rekonstruktionseinrichtung oder wesentliche Teile davon auch in Form von Softwarekomponenten ausgebildet sein. Die Erfindung umfasst somit auch ein Computerprogramm, welches direkt in einen Speicher einer Steuervorrichtung eines Magnetresonanztomographiegeräts ladbar ist, mit Programmcodeabschnitten, um alle Schritte des erfindungsgemäßen Verfahrens auszuführen, wenn das Programm in der Steuervorrichtung ausgeführt wird. Die Steuervorrichtung kann dabei insbesondere auch als System aus mehreren untereinander vernetzten Rechnern bzw. Prozessoren aufgebaut sein. Eine softwaremäßige Realisierung hat den Vorteil, dass auch schon existierende, mit geeigneten programmierbaren Prozessoren und Speichern realisierte Magnetresonanztomographiesysteme durch Implementierung des Programms in geeigneter Weise modifiziert werden können, um in der erfindungsgemäßen Weise zu arbeiten.

Die abhängigen Ansprüche sowie die nachfolgende Beschreibung enthalten besonders vorteilhafte Weiterbildungen und Ausgestaltungen der Erfindung, wobei insbesondere auch die Ansprüche einer Kategorie analog zu den abhängigen Ansprüchen einer anderen Anspruchskategorie weitergebildet sein können. Ebenso können einzelne Merkmale oder Merkmalsgruppen verschiedener Ausführungsbeispiele der Erfindung auch zu neuen Ausführungsbeispielen kombiniert werden.

Die optimale Implementierung eines erfindungsgemäßen Verfahrens zur Durchführung von Sensitivitätsmessungen in 2D-axialen Messungen mit Tischvorschub senkrecht zur Bildebene hängt insbesondere vom verwendeten Sequenztyp der genutzten Bildgebungsmessung ab. Dabei ist wiederum zwischen drei verschiedenen prinzipiellen Familien von bildgebenden Sequenztypen zu unterscheiden.

Zu der ersten Familie gehören die bereits oben im Zusammenhang mit Formel (1) beschriebenen Sequenzen mit kurzem TR, wie TrueFISP oder Protonendichte-gewichtete FLASH Sequenzen. Bei diesen Sequenzen ist auch eine Akquisition der Rohdaten mit den herkömmlichen Verfahren gut möglich, wobei es aber natürlich nicht ausgeschlossen ist, ein erfindungsgemäßes Verfahren einzusetzen.

Zur zweiten Familie gehören Sequenzen mit längerem TR wie T1-gewichtete FLASH-Techniken und insbesondere T2-gewichtete Multi-Shot-Turbo-Spin-Echosequenzen (TSE), bei denen ein TR von 70 ms (für T1-Gewichtung mit FLASH) bis zu mehreren Sekunden (für T2-Gewichtung mit TSE) nötig ist, um einen gewünschten Kontrast zu erzielen bzw. das Signal aufrechtzuerhalten. Insbesondere bei dieser Sequenzfamilie würde das eingangs beschriebene herkömmliche Verfahren sehr ineffizient, da das lange TR bei einem in der MRT typischen Schichtabstand von d = 3 bis 8 mm nach Formel (1) zu einer sehr kleinen Tischgeschwindigkeit führt.

Insbesondere für Sequenztypen dieser Familie werden bei einer bevorzugten Variante des erfindungsgemäßen Verfahrens Teile eines Bildgebungs-Rohdatensatzes, der zur Rekonstruktion von Bilddaten einer Schicht des Bildstapels benötigt wird, in unterschiedlichen Fahrten von der Anfangsposition zur Endposition und/oder der Endposition zur Anfangsposition akquiriert. Mit anderen Worten, es wird hierbei die Rohdatenakquise für eine einzelne Schicht auf mehrere Fahrten verteilt.

Insbesondere, wenn zur Akquisition eines Bildgebungs-Rohdatensatzes für die Bilddaten einer Schicht des Schichtstapels eine mehrfache Anregung der Schicht erforderlich ist, kann dabei bei einer ersten Untervariante des Verfahrens die betreffende Schicht des Schichtstapels pro Fahrt von der Anfangsposition zur Endposition und/oder der Endposition zur Anfangsposition jeweils maximal einmalig angeregt werden, wenn sie sich in einem definierten Positionsbereich relativ zum Magneten-/Gradienten-System befindet. Damit werden also alle Bildgebungs-Rohdaten an der gleichen Position aufgenommen, so dass eventuelle Inhomogenitäten bzw. Linearitätsabweichungen der Magnetfelder alle Daten der Schicht, wie bei einer stationären Aufnahme, in der gleichen Weise beeinflussen. Besonders bevorzugt handelt es sich bei dem definierten Positionsbereich um das Isozentrum des Magneten-/Gradienten-Systems. Bei dieser Variante ist die Zahl der Fahrten mit Datenakquisition somit gleich der Zahl der Anregungen pro Bild bzw. Schicht des Bildstapels.

Bei einer zweiten Untervariante zur Akquisition eines Bildgebungs-Rohdatensatzes für die Bilddaten einer Schicht des Schichtstapels, bei dem eine mehrfache Anregung der Schicht erforderlich ist, kann auch pro Fahrt von der Anfangsposition zur Endposition und/oder der Endposition zur Anfangsposition die Schicht des Schichtstapels (jeweils maximal) mehrmalig angeregt werden. Dies erfolgt vorzugsweise auch wieder jeweils dann, wenn sich die Schicht in einem definierten Positionsbereich im Magneten-/Gradienten-System befindet, wobei die definierten Positionsbereiche, in denen jeweils während einer Fahrt die Anregung der Schicht erfolgt, voneinander beabstandet sind. Vorzugsweise liegen die Positionsbereiche aber auch bei dieser Untervariante noch in der Nähe des Isozentrums, d.h. in einem Bereich mit großer Homogenität und guter Linearität.

Bei einer besonders bevorzugten Ausführungsform dieser Untervariante wird, wenn z. B. zur Akquisition eines Bildes $N_{exc}$ Anregungen nötig sind, pro Fahrt zwischen der Anfangsposition und der Endposition jede Schicht bis zu CEIL ($N_{exc}/N_{sweep}$) mal angeregt, wobei $N_{sweep}$ die Zahl der Fahrten mit Datenakquisition ist und CEIL(.) das Aufrunden zum nächsten ganzzahligen Wert bedeutet. Das effektive im Magneten genutzte Volumen wird dadurch um ungefähr einen Faktor $N_{sweep}$ gegenüber einer konventionellen Akquisitionstechnik mit kontinuierlichem Tischvorschub während der Messung reduziert.

Zu der zweiten Familie werden hier im Übrigen auch sogenannte Single-Shot-Sequenzen gezählt, bei denen alle Daten zu Erstellung eines sogenannten "Primärbildes" nach einer einzigen Anregung ausgelesen werden können, aber mehrere solcher "Primärbilder" jeder Schicht in der Untersuchung akquiriert werden müssen, um daraus die eigentlich gewünschten Bilddaten zu rekonstruieren. Grund für die mehreren Primärbilder können die gewünschte Kombination der Primärbilder zu einem Bild mit verbessertem Signal-zu-Rauschverhältnis oder die Akquisition unterschiedlicher Kontraste -(beispielsweise verschiedenen Diffusions-Wichtungen und/oder Richtungen mit einer Single-Shot-Echoplanar Sequenz). Auch hierfür sind die zuvor beschriebenen (Unter-)Verfahrensvarianten besonders gut geeignet.

Als eine dritte Familie können hier die "echten" Single-Shot-Sequenzen betrachtet werden, bei denen zur Akquisition des Bildgebungs-Rohdatensatzes für die Bilddaten einer Schicht des Schichtstapels (für eine Untersuchung) eine einzige Anregung pro Schicht hinreichend ist. Beispiele hierfür sind das Echoplanar-Imaging (EPI) oder Half-Fourier-Single-Shot-Turbo-Spin-Echo- (HASTE) Sequenzen.

Bei diesen Sequenztypen können im Prinzip wie bei der ersten Familie die Schichtdaten sukzessiv akquiriert werden, also die Daten einer ersten Schicht komplett akquiriert werden, bevor mit der Datenakquisition einer weiteren Schicht begonnen wird. In der Formel (1) ist dabei $N_{exc}$ gleich 1 und das TR (das bei echten Single-Shot-Sequenzen unendlich ist) durch den Zeitraum TS zwischen der Akquisition benachbarter Schichten zu ersetzen. Dieser Zeitraum TS setzt sich wiederrum aus der Zeit TA zur Akquisition der Bildgebungs-Rohdaten einer Einzelschicht und einer Pause TP zwischen der Akquisition benachbarter Schichten zusammen. Somit erhält man die Vorschubgeschwindigkeit gemäß

$$v_{table} = \frac{d}{TA + TP} = \frac{d}{TS} \qquad\qquad (2)$$

Häufig ist die sukzessive Akquisition benachbarter Schichten aber auch in dieser Familie mit erheblichen Nachteilen wie SNR-Verlust bzw. Beeinträchtigung eines gewünschten Kontrastes verbunden. Ursache ist hierfür das sogenannte Übersprechen der Schichten (engl. "cross talk"). Unter Übersprechen der Schichten versteht man dabei die Tatsache, dass jeder selektive Hochfrequenzpuls wegen seiner endlichen Dauer ein unperfektes Anregungsprofil hat. Technisch unvermeidbar beeinflusst jeder Hochfrequenzpuls somit auch Regionen, die sich außerhalb der gewünschten Schicht befinden. Dieser Effekt tritt am stärksten zwischen unmittelbar benachbarten Schichten auf. Wird also eine Nachbarschicht akquiriert, bevor die gestörte Magnetisierung in ihren Gleichgewichtszustand zurückgekehrt ist (was näherungsweise erst nach der vier- bis fünffachen T1-Zeit des Gewebes der Fall ist), so sinkt dadurch die Signalintensität (und damit das Signal-zu-Rausch- Verhältnis) und der Kontrast des Bildes kann sich verändern.

Daher wird in einer weiteren bevorzugten Variante der Erfindung auch dann, wenn zur Akquisition des Bildgebungs-Rohdatensatzes für die Bilddaten einer Schicht des Schichtstapels eine einfache Anregung der Schicht ausreicht (also bei den Sequenztypen der dritten Familie), dafür gesorgt, dass in einer Fahrt von der Anfangsposition zur Endposition und/oder der Endposition zur Anfangsposition verschiedene Schichten des Schichtstapels nur dann angeregt (und deren Bildgebungs-Rohdaten akquiriert) werden, wenn sie voneinander beabstandet sind. Die Bildgebungs-Rohdaten für die anderen Schichten können dann in den weiteren Fahrten akquiriert werden, um so den kompletten Bildgebungs-Rohdatensatz für den ganzen Bildstapel zu erfassen. Durch diese Vorgehensweise wird dafür gesorgt, dass zwischen der Anregung zweier benachbarter Schichten immer genug Wartezeit verbleibt, so dass Übersprecheffekte reduziert oder sogar ganz vermieden werden. Dennoch ist die Methode - insbesondere bei Sequenzen mit längerem TR - effizienter als bei der herkömmlichen Vorgehensweise mit einer langsameren Vorschubgeschwindigkeit. Im Gegensatz zu einer Methode mit einer verschachtelten Aufnahmetechnik kann dabei bevorzugt die Anregung einer Schicht wieder genau dann erfolgen, wenn sie sich in einem definierten Positionsbereich relativ zum Magneten-/Gradienten-System befindet, besonders bevorzugt im Isozentrum des Magneten-/Gradienten-Systems.

Bevorzugt wird pro Fahrt von der Anfangsposition zur Endposition und/oder der Endposition zur Anfangsposition nur jede n-te Schicht angeregt. Für jede Fahrt werden dann z. B. die anzuregenden und zu akquirierenden Schichten (beispielsweise um einen Schichtabstand) verschoben. Es sind dann nach n Fahrten alle Schichten einmalig gemessen. Die Zahl der Fahrten mit Datenakquisition ist somit gleich n. Beispielsweise kann pro Fahrt jede 2-te Schicht angeregt bzw. akquiriert werden, so dass in einer ersten Fahrt alle Schichten mit "gerader Nummer" und in einer zweiten Fahrt genau versetzt alle Schichten mit "ungerader Nummer" angeregt werden.

Zu der genannten dritten Familie werden hier neben "echten" Single-Shot-Sequenzen, bei denen alle k-Raum-Zeilen eines Bildes nach einem einzigen Hochfrequenz-Anregungspuls ausgelesen werden, auch solche Sequenzen mit kurzem TR gezählt, wie Turbo-FLASH, bei denen ein sogenannter selektiver Präparationspuls zur Unterdrückung einer ungewünschten Signalkomponente bzw. zur Erzielung eines gewünschten Kontrastes einmalig pro Bild zum Einsatz kommt. Die Schichtdicke des Präparationspulses wird dabei in der Regel größer gewählt als die Schichtdicke der darauffolgenden Anregungs- bzw. Refokussierungspulse, um eine gleichmäßige Präparation der Magnetisierung zu erreichen. Dies führt bei einer zeitlich kurz aufeinanderfolgenden Erfassung von anatomisch benachbarten Schichten ebenfalls zu den o. g. Übersprech-Problemen. Unabhängig davon, um welchen Sequenztyp es sich handelt, gibt es verschiedene strategische Vorgehensweisen, in welchen Fahrten überhaupt Bildgebungs-Rohdaten akquiriert werden.

Bei einer ersten Variante erfolgt eine Akquisition von Teilen des Bildgebungs-Rohdatensatzes nur bei Fahrten in einer Fahrtrichtung, also z. B. nur bei Fahrten von der Anfangszur Endposition oder jeweils im umgekehrten Fall. Dadurch sind zwar "Leerfahrten" ohne Datenakquisition erforderlich, jedoch kann eine solche Leerfahrt genutzt werden, damit die Magnetisierung vor der nächsten Fahrt wieder in den Gleichgewichtszustand zurückgekehrt. Diese Vorgehensweise hat den Vorteil, dass die Schichten des Bildstapels immer in der gleichen Reihenfolge akquiriert werden. Insbesondere, wenn für eine Akquisition von Bildgebungs-Rohdaten die Schicht mehrfach angeregt werden muss, wird so dafür gesorgt, dass der zeitliche Abstand zwischen zwei aufeinanderfolgenden Anregungen für alle Schichten in etwa gleich ist.

Alternativ kann auch eine Akquisition von Teilen des Bildgebungs-Rohdatensatzes bei Fahrten in beiden Fahrtrichtungen erfolgten, also sowohl bei Fahrten von der Anfangs- zur Endposition als auch von der Endposition zur Anfangsposition. Dadurch kann das Datenakquisitionsverfahren beschleunigt werden. Insbesondere bei einem solchen Verfahren kann, um dafür zu sorgen, dass vor einer Neuanregung die Magnetisierung im Gleichgewichtszustand ist, die Steuerung so erfolgen, dass zwischen zwei Fahrten eine vorgegebene Mindestwartezeit eingehalten wird. D. h. es wird am Ende einer Fahrt eine ausreichend lange Pause zur Relaxation der Magnetisierung eingeführt, bevor die Rückfahrt erfolgt.

Die Erfindung wird im Folgenden unter Hinweis auf die beigefügten Figuren anhand von Ausführungsbeispielen noch einmal näher erläutert. Es zeigen:

Figur 1 eine schematische Darstellung eines Ausführungsbeispiels eines erfindungsgemäßen Magnetresonanztomographiesystems,

Figur 2 eine schematische Darstellung der Durchführung einer erfindungsgemäßen Magnetresonanzmessung gemäß einer ersten Ausführungsvariante,

Figur 3 eine schematische Darstellung der Durchführung einer erfindungsgemäßen Magnetresonanzmessung gemäß einer zweiten Ausführungsvariante,

Figur 4 eine schematische Darstellung der Durchführung einer erfindungsgemäßen Magnetresonanzmessung gemäß einer dritten Ausführungsvariante,

Figur 5 eine schematische Darstellung der Durchführung einer erfindungsgemäßen Magnetresonanzmessung gemäß einer vierten Ausführungsvariante.

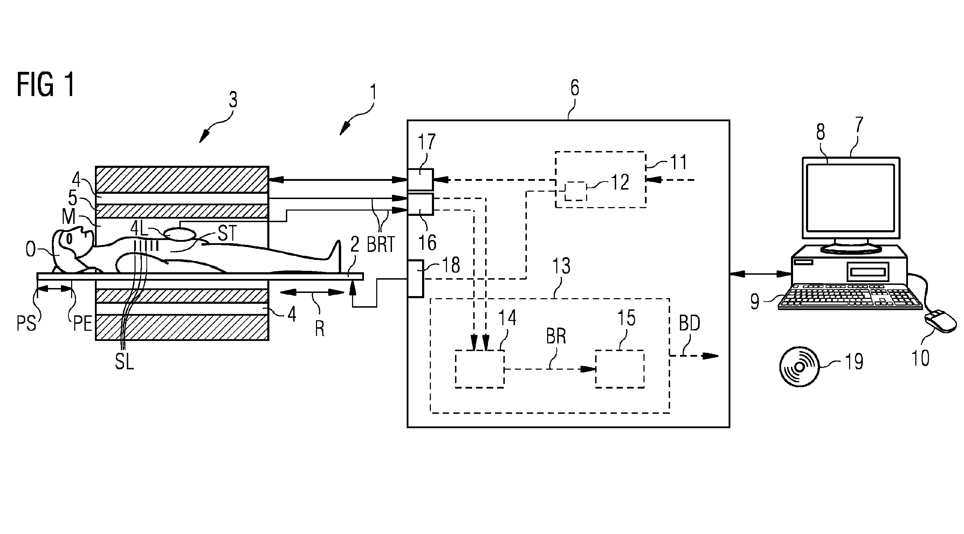

In Figur 1 ist ein Ausführungsbeispiel eines erfindungsgemäßen Magnetresonanztomographiesystems 1 schematisch dargestellt. Das Magnetresonanztomographiesystem 1 umfasst im Wesentlichen einen Magnetresonanztomographen 3 mit einem Magneten-/Gradienten-System 5 (bestehend aus einem hier nicht im einzelnen dargestellten Grundfeldmagnetsystem sowie Gradientenspulen in den verschiedenen Raumrichtungen), mit welchem das für die Magnetresonanzmessung notwendige Magnetfeld sowie die Magnet-Gradientenfelder in einem Messraum M erzeugt werden.

Im Messraum M, auch Patiententunnel genannt, befindet sich ein Tisch 2, auf dem ein Patient bzw. Untersuchungsobjekt O positioniert werden kann. Als ein Antennensystem weist der Magnetresonanztomographen 3 wie üblich eine fest installierte Volumenspule 4 auf. Zudem befinden sich im Messraum am Patienten O positionierten Lokalspulen 4L (von denen hier der Einfachheit halber nur eine dargestellt ist).

Außerdem umfasst das Magnetresonanztomographiesystem 1 eine Steuereinrichtung 6, mit welcher der Tomograph 3 gesteuert wird und Magnetresonanzdaten (insbesondere Bildgebungs-Rohdaten) von dem Tomograph 3 erfasst werden, und ein an die Steuereinrichtung 6 angeschlossenes Terminal 7. Die Steuereinrichtung 6 umfasst ihrerseits eine Ansteuereinheit 11 und eine Rekonstruktionseinrichtung 13 für die Bildgebungs-Rohdaten BR.

Während einer Magnetresonanzmessung werden Teile BRT von Bildgebungs-Rohdatensätzen BR mittels im Messraum M am Patienten O positionierter Lokalspulen 4L (von denen hier nur eine dargestellt ist) und gegebenenfalls der Volumenspule 4 erfasst und nach einer Vorverarbeitung über eine Rohdatenschnittstelle 16 an die Rekonstruktionseinrichtung 13 übergeben, wobei der Tomograph 3 und der Tisch 2 von der Ansteuereinheit 11 über eine Steuerdatenschnittstelle 17 und eine Tischsteuerschnittstelle 18 derart angesteuert werden, dass letztlich ein vollständiger Bildgebungs-Rohdatensatz BR, welcher zur Rekonstruktion von Bilddaten BD für Schichten SL eines zuvor festgelegten, bestimmten Bildstapels ST, welcher ein bestimmtes interessierendes Volumen im Körperinneren eines auf dem Tisch 2 liegenden Patienten O abdeckt, erfasst werden. Die Ansteuereinheit 11 weist hierzu ein Tischansteuermodul 12 auf, welches dafür sorgt, dass erfindungsgemäß während einer solchen Messung der Tisch 2 mit dem Untersuchungsobjekt O mehrfach zwischen einer Anfangsposition PS und einer Endposition PE in einer Tischverfahrrichtung R (welche hier der Längsachse des Magnetresonanztomographen 3 entspricht) hin- und hergefahren wird, und dabei jeweils nur Teile BRT eines Bildgebungs-Rohdatensatzes BR, welcher zur Rekonstruktion von Bilddaten BD für Schichten SL eines Bildstapels ST benötigt wird, in unterschiedlichen Fahrten von der Anfangsposition PS zur Endposition PE und/oder der Endposition PE zur Anfangsposition PS akquiriert werden.

Die Rekonstruktionseinrichtung 13 umfasst hier eine Rohdatensortier- und/oder Zwischenspeichereinheit 14 und eine eigentliche Bildrekonstruktionseinheit 15. Die Rekonstruktionseinrichtung 13 ist mit all diesen Komponenten 14, 15 in Form von Software auf einem geeigneten Prozessor der Steuereinrichtung 6 realisiert. In der Rohdatensortier- und/oder Zwischenspeichereinheit 14 werden die verschiedenen Teile BRT der Bildgebungs-Rohdaten einer Schicht SL aus den verschiedenen Fahrten dieser Schicht SL zugeordnet und so lange zwischengespeichert, bis die Daten für eine Rekonstruktion ausreichen, beispielsweise der komplette Bildgebung-Rohdatensatz BR für diese Schicht akquiriert ist. Dann werden die Bildgebungsrohdaten an die Bildrekonstruktionseinheit 15 übergeben, welche aus den Bildgebungs-Rohdaten BR die Bilddaten BD für die betreffende Schicht rekonstruiert. Dies kann mit einem der üblichen, dem Fachmann bekannten Verfahren erfolgen.

Die Bilddaten BD können dann zum Beispiel auf einem Bildschirm 8 des Terminals 7 grafisch dargestellt werden. Alternativ können die Bildgebungsrohdaten-Rohdaten und/oder Bilddaten auch in einem (nicht dargestellten) Massenspeicher hinterlegt werden.

Neben der grafischen Darstellung der Bilddaten BD kann mit dem Terminal 7, welches neben dem Bildschirm 8 eine Tastatur 9 und eine Maus 10 umfasst, von einem Anwender z. B. ein zu vermessender Bildstapel ST bzw. dessen einzelne Schichten SL vorgegeben werden und weitere Parameter zur Durchführung der erfindungsgemäßen Verfahren bestimmt werden. Üblicherweise erfolgt die Steuerung des Magnetresonanztomographen 3 durch die Steuereinrichtung 6 während der Magnetresonanzmessung vollautomatisch mittels eines Messprotokolls, das der Bediener zuvor aus einer Sammlung von vorgefertigten Messprotokollen ausgewählt und in der Regel modifiziert hat, so dass die von ihm gewünschte Messung durchgeführt wird.

Über das Terminal 7 kann auch die Software für die Steuereinrichtung 6, insbesondere für die Bildrekonstruktionseinrichtung 13, in die Steuereinrichtung 6 geladen werden. Diese Software der Steuereinrichtung 6 kann dabei auch die erfindungsgemäßen Verfahren umfassen. Es ist dabei auch möglich, dass ein erfindungsgemäßes Verfahren in einer Software enthalten ist, welche in dem Terminal 7 abläuft. Unabhängig davon, in welcher Software das erfindungsgemäße Verfahren enthalten ist, kann die Software auf einer DVD 19 oder einem anderen Datenträger gespeichert sein, so dass diese Software dann von dem Terminal 7 von der DVD 19 gelesen und entweder in die Steuereinrichtung 6 oder in eine Recheneinheit des Terminals 7 selbst kopiert werden kann.

Die Steuerdatenschnittstelle 17, die Rohdatenschnittstelle 16 und die Tischsteuerschnittstelle 18 sind hier sehr vereinfacht jeweils nur als ein Block dargestellt. Tatsächlich bestehen diese Schnittstellen aus einer Vielzahl von einzelnen Komponenten. Beispielsweise umfasst die Steuerdatenschnittstelle 17 einen oder mehrere Sendekanäle mit entsprechenden Hochfrequenzverstärkern, um HF-Pulse mit der benötigten Stärke und Pulsform in die Volumenspule 4 einspeisen zu können, sowie geeignete (Gradienten-) Schnittstellen, um die Gradientenspulen mit den passenden Gradientenpulsen zu versorgen. Ebenso weist die Rohdatenschnittstelle 16 eine Vielzahl von Empfangskanälen für die Volumenspule 4 und die verschiedenen Lokalspulen 4L auf.

Es wird an dieser Stelle darauf hingewiesen, dass ein solches Magnetresonanztomographiesystem 1, insbesondere die Steuereinrichtung 6, noch eine Vielzahl weiterer Komponenten aufweisen kann, beispielsweise Schnittstellen zum Anschluss an ein Netzwerk, um die Rohdaten und/oder die rekonstruierten Bilddaten auch an andere Stationen zu übergeben etc. Ebenso kann auch der Magnetresonanztomograph 3 anders als hier dargestellt aufgebaut sein, beispielsweise als seitlich offener Tomograph. Da der grundsätzliche Aufbau von Magnetresonanztomographiesystemen dem Fachmann aber bekannt ist, wurde aus Gründen der Übersichtlichkeit darauf verzichtet, all diese Komponenten und Varianten in Figur 1 darzustellen und hier näher zu erläutern.

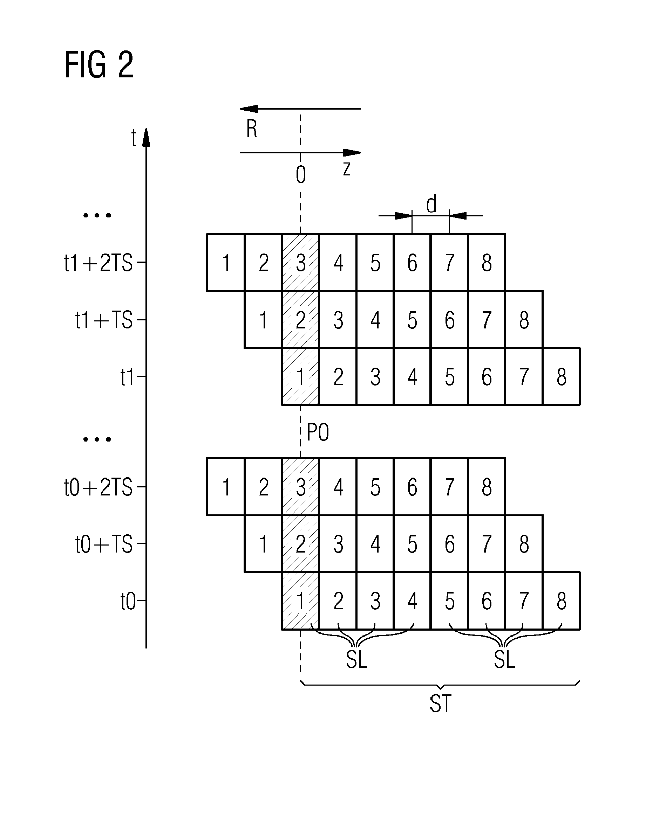

Im Folgenden werden anhand der Figuren 2 bis 5 beispielhaft verschiedene bevorzugte Verfahren erläutert. Bei allen nachfolgend beschriebenen Verfahren erfolgen die Bildgebungsmessungen in Form von axialen 2D-Messungen mit einem Tischvorschub senkrecht zur Bildebene. Das heißt, die Ausleserichtung ist senkrecht zur Tischvorschubrichtung R.

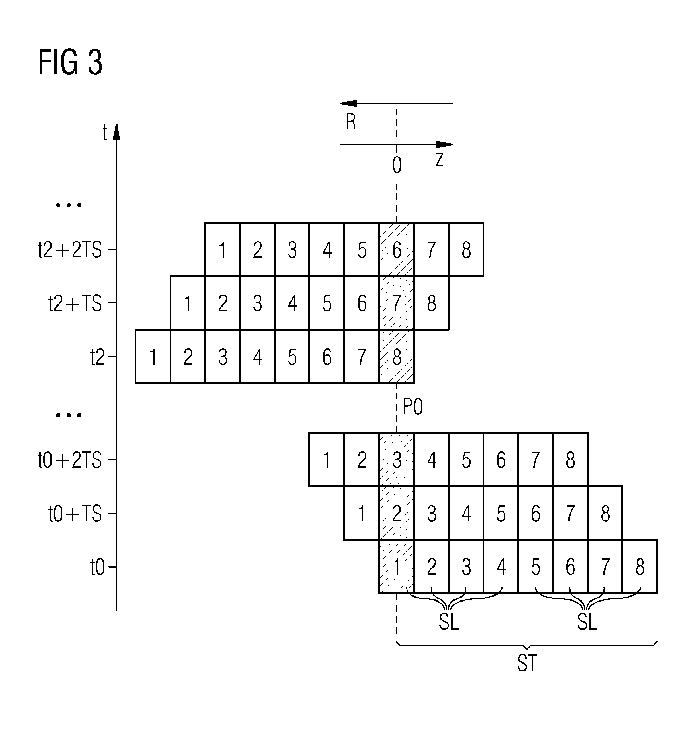

Abbildung 2 zeigt eine erste Ausführungsform der Erfindung. Der Untersuchungsbereich besteht hier, wie auch bei den weiteren Beispielen in den Figuren 3 bis 5, aus einem Schichtstapel ST mit $N_{slc} = 8$ Schichten SL. Die Schichten sind in den Figuren 2 bis 5 jeweils mit ganzen Zahlen 1 bis 8 durchnummeriert, um sie einzeln benennen zu können. Die Reihenfolge ist aber beliebig. Der Abstand zwischen zwei benachbarten Schichten SL dieses Schichtstapels ST wird hier mit d bezeichnet. Zur Erstellung eines Bildes muss jede dieser Schichten SL mindestens $N_{exc}$ mal angeregt und das in Folge einer Anregung emittierte Signal jeweils ortskodiert und ausgelesen werden.

Dazu wird in der ersten Ausführungsform der Tisch 2 derart angefahren, dass sich zu einem ersten Messzeitpunkt t0 eine erste Schicht "1" im Isozentrum (im Positionsbereich bzw. an der Position P0 entsprechend z = 0) des Magneten-/Gradienten-Systems 5 (im Folgenden nur kurz als "Magnet" bezeichnet) befindet. Diese Schicht wird zum Zeitpunkt t0 gemessen. Gemessen bedeutet hier und im Folgenden, dass die betreffende Schicht mit einem Hochfrequenzpuls angeregt und das in Folge des gestörten Gleichgewichtszustandes von den Spins dieser Schicht emittierte Magnetresonanzsignal ortskodiert und ausgelesen wird. Das ist ein Vorgang endlicher Dauer, welche im Folgenden als Akquisitionsintervall TA bezeichnet wird. Einem solchen Akquisitionsintervall TA ist ein Zeitpunkt (hier t0) zugeordnet, der z. B. mit dem Anfang oder der Mitte des Akquisitionsintervalls TA zusammen fällt.

In der ersten Ausführungsform ist der Betrag der Tischgeschwindigkeit $v_1$ konstant und beträgt

$$|v_1| = \frac{d}{TS} = \frac{d}{TA + TP} \qquad (3)$$

Dabei ist d der Abstand zwischen benachbarten Schichten. TS ist der Zeitraum zwischen dem Messen benachbarter Schichten. Diese Zeit setzt sich wiederum aus der Akquisitionsdauer TA pro Schicht und einer optionalen Pause TP zwischen dem Messen benachbarter Schichten zusammen (siehe auch Formel (2)).

Der Schichtabstand d wird in der Regel vom Anwender vorgegeben. Die Akquisitionsdauer ist meist implizit durch eine Reihe von vom Anwender vorgegebenen Parametern festgelegt (wie einer Echo-Zeit, Auflösung, Auslesebandbreite, ...). Die Pause TP ist im Prinzip frei wählbar.

Die maximale Tischgeschwindigkeit und damit die höchste Effizienz der Methode kann mit TP = 0 erreicht werden. Trotzdem gibt es zahlreiche Gründe, weshalb bevorzugt ein TP ungleich Null bevorzugen werden kann:

- Einhaltung einer maximalen bauartbedingten Geschwindigkeit
- Einhaltung der Patientensicherheit, u. a. limitierte spezifische Absorptionsraten
- Erhöhung des Patientenkomforts
- Vermeidung von Übersprechen benachbarter Schichten
- Begrenzung physikalischer Effekte infolge der bewegten Liege während der Datenakquisition.

Die Anfangsposition der Liege wird hier bevorzugt derart gewählt, dass sich die erste Schicht zum Zeitpunkt t0 im Isozentrum des Magneten befindet. Wenn $z_0$ die zu diesem Zeitpunkt t0 zugehörige Liegenposition ist (Schicht "1" im Isozentrum), dann ergibt sich daraus die Anfangsposition PS der Liege wie folgt:

$$PS = z_0 + v_1^2/a_1, \qquad (4)$$

wobei $a_1$ hier der Betrag einer konstanten Liegenbeschleunigung während der Beschleunigungsphase der Liege ist.

Die Endposition PE der Liege ist dann:

$$\texttt{PE} = \texttt{z}_0 - \texttt{d} \cdot \texttt{N}_{\texttt{slc}} - \texttt{v}_1{}^2/\texttt{a}_2 \qquad\qquad (5)$$

Dabei ist $a_2$ hier der Betrag einer konstanten Liegenbeschleunigung während der Abbremsphase der Liege.

In den in den Figuren 2 bis 5 gezeichneten Beispielen ist die Akquisitionsreihenfolge jeweils absteigend (weshalb der Fahrtrichtungspfeil R von größeren z-Positionen zu kleineren z-Positionen, also von positiven zu negativen z-Werten bezogen auf das Isozentrum bei z = 0, zeigen soll). Daraus ergeben sich hier, ohne Beschränkung der Allgemeinheit der Erfindung, die negativen Vorzeichen in der Gleichung.

Die Zahl der Fahrten mit Datenakquisition ist in dieser ersten Ausführungsform gemäß Figur 2 gleich der Zahl der Anregungen. Dabei kann die Datenakquisition entweder in einer Fahrtrichtung (also nur bei Fahrten von der Anfangs- zur Endposition, wie in Figur 2, oder umgekehrt) oder in beiden Fahrtrichtungen erfolgen. Diese zweite Alternative ist in Figur 3 schematisch dargestellt.

In der ersten Variante gemäß Figur 2 (eine Fahrtrichtung) wird der Tisch nach Erreichen der Endposition PE zur Anfangsposition PS zurückgefahren. Die Rückfahrt erfolgt aus Effizienzgründen in der Regel mit höherer Geschwindigkeit $v_{return}$ als die Fahrten mit Datenakquisition.

Die Repetitionszeit TR (die Zeit zwischen sukzessiver Anregung einer bestimmten Schicht) ist in dieser ersten Variante für alle Schichten gleich und beträgt mindestens:

$$TR \geq N_{slc} \times TS + \left(\frac{d \cdot N_{slc}}{|v_{return}|}\right) + \frac{|v_1| + |v_{return}|}{a_1} + \frac{|v_1| + |v_{return}|}{a_2} \qquad\qquad (6)$$

Dabei ist der erste Term die Zeit für die Fahrt mit Datenakquisition, der zweite Term die Zeit für die Rückfahrt, der dritte Term die Zeit für die zwei Beschleunigungsphasen und der vierte Term die Zeit für die zwei Abbremsphasen. Der Grund für das "$\geq$" - Zeichen ist, dass sich die Zeit durch das Einfügen von Pausen (bevorzugt nach Erreichen der End- oder Anfangsposition) beliebig verlängern lässt. In der Regel ist dies in dieser Variante aber nicht nötig, da die Magnetisierung schon während der minimalen Repetitionszeit nahezu vollständig relaxieren kann (was ungefähr nach dem Fünffachen der T1-Zeit des Gewebes der Fall ist). Die Repetitionszeit kann deshalb bei dieser Methode als unendlich angesehen werden, was für die meisten relevanten Anwendungen (T2-TSE, DW-EPI) ein Vorteil ist.

Bei der zweiten Variante der ersten Ausführungsform (Datenakquisition während Hin-und Rückfahrt gemäß Figur 3) variiert die Repetitionszeit von Schicht zu Schicht. Dies hat keinen Einfluss auf den Bildkontrast, sofern von einer vollständigen Relaxation der Magnetisierung während zweier aufeinanderfolgender Anregungen ausgegangen werden kann. Um dies auch für die Schichten, die z. B. in der Nähe der Endposition lokalisiert sind, zu erzwingen, kann vorzugsweise in dieser Variante eventuell eine Pause zwischen den Fahrten (Hin-und Rückfahrt) eingelegt werden.

Eine zweite grundsätzliche Ausführungsform der Erfindung kann als eine Art "Hybridtechnik" einer beliebigen Multi-Shot-Akquisitionstechnik mit kontinuierlichem Tischvorschub im Stand der Technik und der erfindungsgemäßen Technik mit nur einer Anregung pro Fahrt angesehen werden. Bei einer solchen "Hybridtechnik" kann die Zahl der Fahrten $N_{sweep}$ mit Datenakquisition beliebig zwischen 1 und der Zahl der Anregungen pro Bild gewählt werden. Die Zahl der Anregungen pro Fahrt ist dann bis zu:

$$N_{exc\,per\,sweep} = CEIL\left(\frac{N_{exc}}{N_{sweep}}\right) . \qquad\qquad (7)$$

Dabei bedeutet CEIL(.), dass der Ausdruck in Klammern auf den nächsten ganzzahligen (engl. "integer") Wert aufzurunden ist. Entsprechend kann statt der Zahl der Fahrten die Zahl der Anregungen pro Fahrt $N_{exc\,per\,sweep}$ zwischen 1 und Gesamtzahl der Anregungen $N_{exc}$ vorgegeben werden. Die Zahl der Fahrten mit Datenakquisition ist dann:

$$N_{sweep} = CEIL\left(\frac{N_{exc}}{N_{exc\,per\,sweep}}\right) . \qquad\qquad (8)$$

Im Spezialfall $N_{sweep} = 1$ (nur eine Fahrt) geht diese Technik über in die konventionelle Technik. Im Spezialfall $N_{exc} = N_{sweep}$ ist die Technik identisch mit der ersten Ausführungsform der Erfindung. Für die verbleibenden Fälle gilt, dass die Zahl der Anregungen pro Fahrt ungefähr um einen Faktor $N_{sweep}$ reduziert ist. Entsprechend ist der effektive Scanbereich (also der Bereich im Scanner, in dem Daten akquiriert werden) in z-Richtung ungefähr um einen Faktor $N_{sweep}$ bezüglich der konventionellen Technik reduziert. Die eingangs erwähnten Probleme mit der variierenden Scanposition sind entsprechend reduziert.

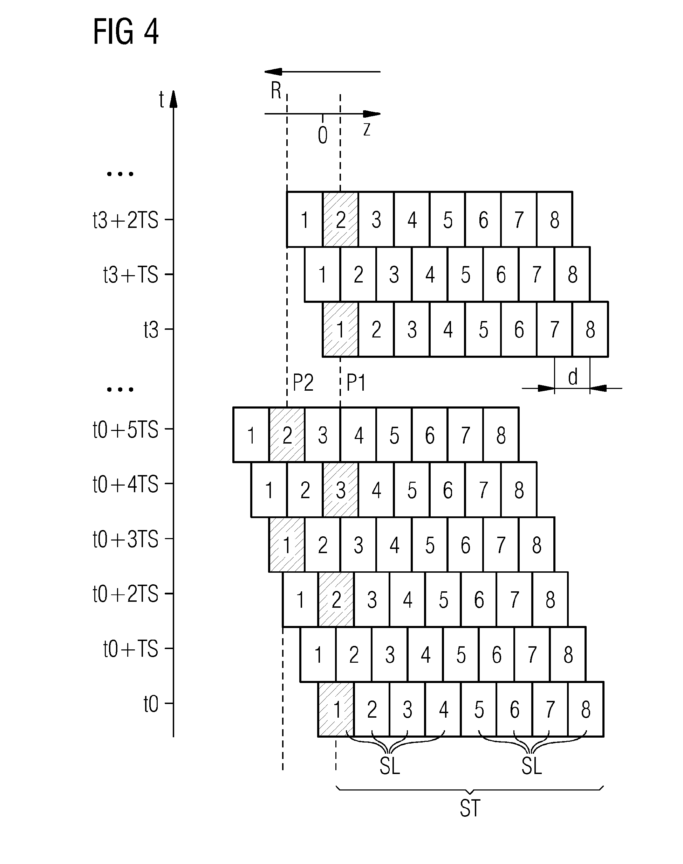

Figur 4 zeigt ein einfaches Beispiel für diese Ausführungsform. Hier erfolgen zwei Anregungen pro Fahrt. Es gibt zwei Scanpositionen P1, P2 (Positionsbereiche, in denen Rohdaten akquiriert werden) im Magnetresonanztomographen (Scanner), die bevorzugt symmetrisch um das Isozentrum des Magneten verteilt sind. Eine Schicht wird jeweils gemessen, wenn sie sich am Ort einer der zwei Scanpositionen P1, P2 befindet.

Zum Zeitpunkt t0 befindet sich die Schicht "1" an einer ersten Scanposition P1 und wird ein erstes Mal gemessen. Zum nächsten Messzeitpunkt t0+TS befindet sich die Schicht, die links an den zu messenden Schichtstapel angrenzt, an der zweiten Scanposition P2. Deren Daten werden nicht gebraucht. Eine Anregung ohne Datenerfassung kann trotzdem Sinn machen, da diese Anregung wegen des Übersprechens der Schichten auch den Kontrast der Schicht "1" beeinflusst. Wieder ein Zeitintervall TS später befindet sich die Schicht "2" an der ersten Scanposition P1 und wird gemessen. Zum nächsten Messzeitpunkt t0 + 3TS hat die Schicht "1" die zweite Scanposition P2 erreicht und wird ein zweites Mal gemessen.

Der Tischvorschub pro Zeitintervall TS und damit die Vorschubgeschwindigkeit $v_2$ ist gegenüber der Ausführung gemäß Figur 2 halbiert, allgemein:

$$|v_2| = \frac{d}{N_{exc\ per\ sweep} \cdot TS} . \qquad (9)$$

Die Zahl der Fahrten mit Datenakquisition ist annähernd um den Faktor $N_{exc\ per\ sweep}$ reduziert.

Die restlichen Anregungen der Schicht "1" erfolgen während der zweiten Fahrt, beginnend zum Zeitpunkt "t3". Die Schicht "1" befindet sich wieder an der ersten Scanposition "P1". In der in Figur 3 gezeichneten Ausführungsform erfolgt die Datenakquisition nur in der "Vorwärtsrichtung".

Bei dieser Ausführungsform variiert die Repetitionszeit TR. Zwischen den verschiedenen Anregungen einer bestimmten Schicht während einer Fahrt vergeht im Beispiel die Zeit 3TS, während zwischen der letzten Anregung der Schicht während der ersten Fahrt und ersten Anregung der Schicht während der zweiten Fahrt die Zeit t3-t0 vergeht, die im Allgemeinen wesentlich länger ist als 3TS. Eventuellen Bildqualitätseinbußen infolge der variierenden TR kann aber durch weitere Maßnahmen (wie z. B. eine extra Anregung pro Fahrt zur Erreichung des stationären Gleichgewichtszustandes, einen Steady-State-Präparationspuls vor der ersten Anregung einer Fahrt, durch ein Messen von peripheren k-Raum-Zeilen während der ersten Anregung einer Fahrt etc.) begegnet werden.

Die bisher beschriebenen Ausführungsformen gemäß den Figuren 2 bis 4 sind alle Anwendungen der oben definierten zweiten Familie von Sequenztypen ("Multi-Shot-Sequenzen").

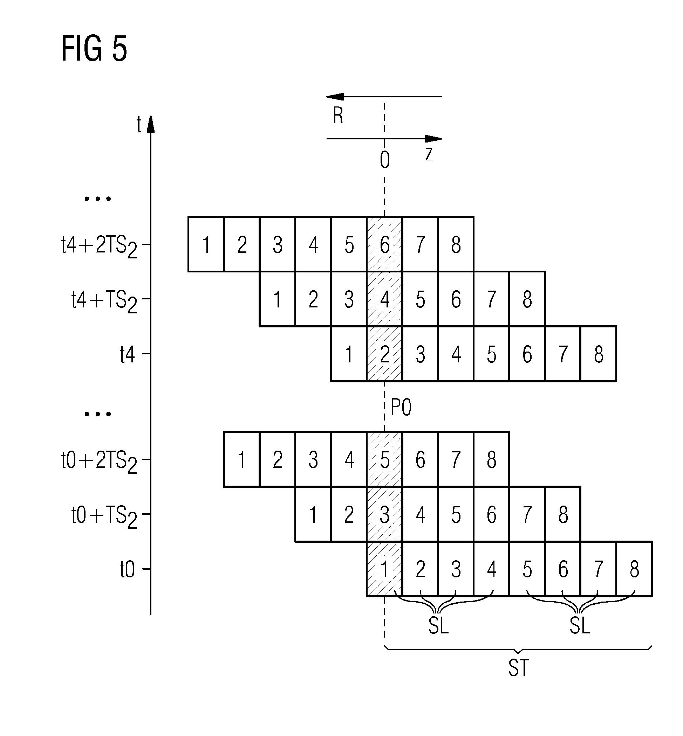

In Figur 5 ist schematisch eine Anwendung der Erfindung für Sequenztypen der dritten Familie gezeigt. Ziel dieser Ausführungsform ist es, den Cross-Talk zwischen den Schichten zu verringern, ohne die Nachteile einer Verschachtelung (engl. "interleaving") in Kauf zu nehmen. Dazu wird in jeder Fahrt mit Datenakquisition nur jede $N_{sweep}$-te Schicht gemessen. Die verbleibenden (in der ersten Fahrt nicht gemessenen) Schichten werden in ($N_{sweep}$-1) weiteren Fahrten gemessen. In jeder der verbleibenden Fahrten wird wiederum nur jede $N_{sweep}$-te Schicht gemessen. Die Startposition wird zwischen aufeinanderfolgenden Fahrten (mit Datenakquisition in einer Richtung) z. B. um einen Schichtabstand d inkrementiert.

Figur 5 zeigt hierzu ein sehr einfaches Beispiel. Hier werden die Schichten in zwei Fahrten akquiriert. In der ersten Fahrt werden zunächst die "ungeraden" Schichten angeregt und gemessen, beginnend mit der Schicht "1" zum Zeitpunkt t0. Die Messung der "geraden" Schichten erfolgt in einer zweiten Fahrt, beginnend mit der Messung der Schicht "2" zu Zeitpunkt t4.

Wird die Zeit TA + TP zwischen der Messung zweier aufeinanderfolgender Schichten festgehalten, dann ist die Tischgeschwindigkeit $v_4$ gegenüber der konventionellen Technik (Formel (2)) um einen Faktor $N_{sweep}$ erhöht:

$$V_4 = \frac{N_{sweep} \cdot d}{TA+TP} = \frac{N_{sweep} \cdot d}{TS} . \qquad (10)$$

Wie eingangs erwähnt, ist das Übersprechen zwischen unmittelbar benachbarten Schichten am größten und klingt mit der Zeit zwischen der Akquisition dieser Schichten exponentiell ab. Die Zeit $\Delta t_{NN}$ zwischen der Akquisition zweier unmittelbar benachbarter Schichten ist hier von TA+TP (in Figur 5 gilt: TA+TP=$TS_2$) mindestens auf die Zeit zwischen zwei aufeinanderfolgenden Fahrten erhöht (in Figur 5 gilt: $\Delta t_{NN}$ = t4-t0). Die Zeit $\Delta t_{NN}$ beträgt bei einer Rohdatenakquisition in einer Richtung mindestens:

$$\Delta t_{NN} > \frac{N_{slc} d}{v_4} = \frac{N_{slc}}{N_{sweep}} TS, \quad \text{für} \quad N_{sweep} > 1 \,. \tag{11}$$

$N_{slc}$ bezeichnet dabei wiederum die Gesamtzahl der Schichten und $N_{sweep}$ die Gesamtzahl der Fahrten mit Datenakquisition. Mehr als zwei Fahrten ($N_{sweep} > 2$) sind nur dann nötig und sinnvoll, wenn das Übersprechen zwischen übernächsten Nachbarschichten nicht vernachlässigbar ist.

Das erfindungsgemäße Verfahren hat je nach Ausführungsform eine Reihe von Vorteilen, die hier noch einmal zusammengefasst werden:

Gegenüber der "klassischen" Technik, bei der die Schichten sukzessive während einer einzigen Fahrt im Isozentrum gemäß Formel (1) akquiriert werden, ergibt sich ein Effizienzgewinn, insbesondere für Aufnahmetechniken mit langer Repetitionszeit. "Effizienzgewinn" bedeutet dabei Reduktion der Untersuchungsdauer. Die Akquisitionszeit ist bei der klassischen Technik bei gegebener Länge des Untersuchungsbereichs direkt proportional zur Liegengeschwindigkeit. Diese ist bei der klassischen Technik durch die Parameter, die Bildkontrast und Auflösung (TR, Nexc, d) bestimmen, quasi vorgegeben. Bei der erfindungsgemäßen Technik ist dagegen die Effizienz proportional zu der Tischgeschwindigkeit und der Zahl der Fahrten. Die Tischgeschwindigkeit ist dabei quasi nur durch die Akquisitionszeit pro Anregung TA nach oben begrenzt und damit für solche Akquisitionstechniken, bei denen eine vollständige Relaxation der Magnetisierung zwischen den Anregungen gewünscht ist, unabhängig von den Auflösung und kontrastbestimmenden Parametern frei wählbar.

Im Stand der Technik sind verschiedene Verschachtelungstechniken mit kontinuierlichem Tischvorschub bekannt, die es erlauben, die Effizienz gegenüber der "klassischen" Aufnahmetechnik zu steigern. Die Vorteile der erfindungsgemäßen Technik gegenüber diesen bekannten Verschachtelungstechniken ergeben sich aus deren Nachteilen: Bei der verschachtelten Technik werden die Schichten nicht mehr fortlaufend im Isozentrum der Anlage akquiriert. Bei den Multi-Shot-Techniken kann die Anregung an verschieden Orten innerhalb der MR-Anlage infolge der geänderten Aufnahmebedingungen zu Bildartefakten und damit zu einer Verschlechterung der Bildqualität führen. Zusätzlich kommt es bei einigen Verschachtelungstechniken zu Zwischenbild-Artefakten, da benachbarte Schichten unterschiedlich akquiriert werden. Bei den Single-Shot-Techniken führt die Verschachtelung ebenfalls zu Zwischenbild-Artefakten, da benachbarte Schichten notwendig an verschieden Orten der MR-Anlage aufgenommen werden. In der Regel variiert auch die Zeit zwischen der Aufnahme einer bestimmten Schicht und der Akquisition ihrer Nachbarschicht. Folge ist eine Kontrastvariation zwischen den Schichten. Die erfindungsgemäße Technik erlaubt dagegen, je nach Ausführungsform, eine Effizienzsteigerung völlig ohne diese Nachteile oder zumindest mit einer starken Reduzierung der nachteiligen Effekte.

Bei den bevorzugten Anwendungen der neuen Technik (T2-Bildgebung mit Turbo-Spin-Sequenzen, diffusionsgewichtete Bildgebung mit Spin-Echo EPI) ist die gewünschte TR-Zeit häufig unendlich. TR unendlich bedeutet, dass eine komplette Relaxation der Magnetisierung zwischen den Anregungen gewünscht ist. Bei der erfindungsgemäßen Technik ist für die genannten Anwendungen die TR-Zeit gleich der Zeit zwischen den Fahrten. Diese kommt häufig der gewünschten vollständigen Relaxation sehr nahe. Bei üblicher Ausdehnung des Untersuchungsbereiches in Tischvorschub-Richtung (>= 1 m) und Tischgeschwindigkeiten (<= 50 mm/s) ist TR also größer oder gleich 2s. Dabei ist die Zeit für Anfahren, Abbremsen, Rückfahrt noch nicht einberechnet. Häufig ergibt sich aber wegen eines größeren Untersuchungsbereiches bzw. kleinerer Tischgeschwindigkeit (SAR, ...) ein TR in der Größenordnung von 10s. Bei diesem TR ist das meiste Gewebe nahezu vollständig relaxiert. In diesem Fall ergibt sich zusätzlich ein SNR- und/oder Kontrastgewinn.

Bei den Verschachtelungstechniken im Stand der Technik ist der Verfahrweg der Liege häufig größer als der Untersuchungsbereich, von dem Bilder berechnet werden. Dies lässt sich z. B. an dem Beispiel in Figur 4 ersehen. Zum Beginn der Messung (Zeitpunkt t0) befindet sich die erste Schicht "1" an der ersten Scanposition "P1". Die Messung ist beendet, wenn die letzte Schicht "8" vollständig gemessen ist und die zweite Scanposition "P2" gerade durchlaufen hat. Dann ist die Liege insgesamt um die Strecke |P2-P1| + d · $N_{slc}$ (Schichtanzahl $N_{slc}$ = 8 in Figur 3), also um die Strecke |P2-P1| größer als der Bereich, aus dem Bildgebungs-Rohdaten akquiriert werden. Damit ist die maximale Ausdehnung des Untersuchungsbereiches in z-Richtung (Tischvorschubrichtung) kleiner als der bauartbedingte maximale Verfahrweg der Liege. Bei der erfindungsgemäßen Methode ist der maximale Untersuchungsbereich in z-Richtung, je nach Ausführungsvariante der Erfindung, gar nicht (abgesehen von dem Verfahrweg, der zum Abbremsen und Beschleunigung der Liege am Anfang und am Ende einer Fahrt benötigt wird) oder zumindest weniger gegenüber dem maximalen Verfahrweg der Liege reduziert.

Es wird abschließend noch einmal darauf hingewiesen, dass es sich bei den zuvor beschriebenen detaillierten Verfahren und Aufbauten um Ausführungsbeispiele handelt und dass das Grundprinzip auch in weiten Bereichen vom Fachmann variiert werden kann, ohne den Bereich der Erfindung zu verlassen, soweit er durch die Ansprüche vorgegeben ist. Auch wenn die Erfindung vorstehend anhand eines Magnetresonanztomographiesystems im medizinischen Bereich beschrieben wurde, ist die Erfindung auch in wissenschaftlichen und/oder industriell genutzten Magnetresonanztomographiesystemen einsetzbar. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können. Ebenso schließt der Begriff "Einheit" oder "Modul" nicht aus, dass diese aus mehreren Komponenten besteht, die gegebenenfalls auch räumlich verteilt sein können.

**Patentansprüche**

1. Verfahren zur Erzeugung von Bilddaten (BD) eines Untersuchungsobjekts (O) mittels eines Magnetresonanztomographen (3),
   bei dem während einer Magnetresonanzmessung das Untersuchungsobjekt (O) relativ zu einem Magneten-/Gradienten-System (5) des Magnetresonanztomographen (3) mehrfach zwischen einer Anfangsposition (PS) und einer Endposition (PE) hin- und hergefahren wird und dabei jeweils Teile (BRT) eines Bildgebungs-Rohdatensatzes (BR), welcher zur Rekonstruktion von Bilddaten (BD) für Schichten (SL) eines Bildstapels (ST) benötigt wird, in unterschiedlichen Fahrten von der Anfangsposition (PS) zur Endposition (PE) und/oder der Endposition (PE) zur Anfangsposition (PS) akquiriert werden.

2. Verfahren nach Anspruch 1, **dadurch gekennzeichnet, dass** Teile (BRT) eines Bildgebungs-Rohdatensatzes (BR), welcher zur Rekonstruktion von Bilddaten (BD) einer Schicht (SL) benötigt wird, in unterschiedlichen Fahrten von der Anfangsposition (PS) zur Endposition (PE) und/oder der Endposition (PE) zur Anfangsposition (PS) akquiriert werden.

3. Verfahren nach Anspruch 2, **dadurch gekennzeichnet, dass** zur Akquisition eines Bildgebungs-Rohdatensatzes (BR) für die Bilddaten (BD) einer Schicht (SL) des Schichtstapels (ST) (ST) eine mehrfache Anregung der Schicht (SL) erforderlich ist und in einer Fahrt von der Anfangsposition (PS) zur Endposition (PE) und/oder der Endposition (PE) zur Anfangsposition (PS) die Schicht (SL) des Schichtstapels (ST) einmalig angeregt wird, wenn sie sich in einem definierten Positionsbereich (P0, P1, P2) relativ zum Magneten-/Gradienten-System befindet.

4. Verfahren nach Anspruch 2, **dadurch gekennzeichnet, dass** zur Akquisition eines Bildgebungs-Rohdatensatzes (BR) für die Bilddaten (BD) einer Schicht (SL) des Schichtstapels eine mehrfache Anregung der Schicht (SL) erforderlich ist und in einer Fahrt von der Anfangsposition (PS) zur Endposition (PE) und/oder der Endposition (PE) zur Anfangsposition (PS) die Schicht (SL) des Schichtstapels (ST) mehrmalig angeregt wird.

5. Verfahren nach Anspruch 1, **dadurch gekennzeichnet, dass** zur Akquisition des Bildgebungs-Rohdatensatzes (BR) für die Bilddaten (BD) einer Schicht (SL) des Schichtstapels eine einfache Anregung der Schicht (SL) ausreicht und in einer Fahrt von der Anfangsposition (PS) zur Endposition (PE) und/oder der Endposition (PE) zur Anfangsposition (PS) verschiedene Schichten (SL) des Schichtstapels (ST) nur dann angeregt werden, wenn sie voneinander beabstandet sind, wobei die Anregung einer Schicht (SL) vorzugsweise genau dann erfolgt, wenn sie sich in einem definierten Positionsbereich (P0, P1, P2) relativ zum Magneten-/Gradienten-System (5) befindet.

6. Verfahren nach Anspruch 5, **dadurch gekennzeichnet, dass** in einer Fahrt von der Anfangsposition (PS) zur Endposition (PE) und/oder der Endposition (PE) zur Anfangsposition (PS) nur jede n-te Schicht (SL) angeregt wird.

7. Verfahren nach einem der Ansprüche 1 bis 6, **dadurch gekennzeichnet, dass** eine Akquisition von Teilen (BRT) des Bildgebungs-Rohdatensatzes (BR) nur bei Fahrten in einer Fahrtrichtung erfolgt.

8. Verfahren nach einem der Ansprüche 1 bis 6, **dadurch gekennzeichnet, dass** eine Akquisition von Teilen (BRT) des Bildgebungs-Rohdatensatzes (BR) bei Fahrten in beiden Fahrtrichtungen erfolgt.

9. Verfahren nach einem der Ansprüche 1 bis 8, **dadurch gekennzeichnet, dass** zwischen zwei Fahrten eine vorgegebene Mindestwartezeit eingehalten wird.

10. Magnetresonanztomographiesystem (1) zur Erzeugung von Bilddaten (BD) eines Untersuchungsobjekts (O) mit

- einem Magnetresonanztomographen (3) mit einem Magneten-/Gradienten-System (5),
- einer Steuervorrichtung (6), welche ausgebildet ist, um den Magnetresonanztomographen (3) bei einer Magnetresonanzmessung so anzusteuern, dass das Untersuchungsobjekt (O) relativ zu einem Magneten-/Gradienten-System (5) des Magnetresonanztomographen (3) mehrfach zwischen einer Anfangsposition (PS) und einer Endposition (PE) hin- und hergefahren wird und dabei jeweils Teile (BRT) eines Bildgebungs-Rohdatensatzes (BR), welcher zur Rekonstruktion von Bilddaten (BD) für Schichten (SL) eines Bildstapels (ST) benötigt wird, in unterschiedlichen Fahrten von der Anfangsposition (PS) zur Endposition (PE) und/oder der Endposition (PE) zur Anfangsposition (PS) akquiriert werden.

11. Magnetresonanztomographiesystem (1) nach Anspruch 10, mit einer Rekonstruktionseinrichtung (13), welche ausgebildet ist, um auf Basis der Bildgebungs-Rohdaten (BR) Bilddaten (BD) der Schichten (SL) des Bildstapels (ST) zu rekonstruieren.

12. Computerprogrammprodukt, welches direkt in einen Speicher eines Steuervorrichtung (6) eines Magnetresonanztomographiesystems (1) ladbar ist, mit Programmcodeabschnitten, um alle Schritte eines Verfahrens nach einem der Ansprüche 1 bis 9 auszuführen, wenn das Programm in der Steuervorrichtung (6) ausgeführt wird.

FIG 1

FIG 2

FIG 3

# FIG 4

# FIG 5

Europäisches
Patentamt

European
Patent Office

Office européen
des brevets

**EUROPÄISCHER RECHERCHENBERICHT**

Nummer der Anmeldung

EP 15 15 4066

| | **EINSCHLÄGIGE DOKUMENTE** | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| X | WO 2010/001285 A1 (KONINKL PHILIPS ELECTRONICS NV [NL]; PHILIPS INTELLECTUAL PROPERTY [DE] 7. Januar 2010 (2010-01-07) * Seite 11, Zeile 5 - Seite 12, Zeile 8 * ----- | 1,7,8, 10-12 | INV. G01R33/563 ADD. G01R33/483 G01R33/565 |
| X | US 2003/011369 A1 (BRITTAIN JEAN H [US] ET AL) 16. Januar 2003 (2003-01-16) | 1-4,8-12 | |
| Y | * Absätze [0050], [0053], [0073]; Ansprüche 1,3 * ----- | 5,6 | |
| Y | LUDWIG U.A. ET AL.: "Multiple Volume Coverage in Cintinuously Moving Table Acquisitions Applied to Free Breathing STIR imaging", PROCEEDINGS OF THE INTERNATIONAL SOCIETY FOR MAGNETIC RESONANCE IN MEDICINE, 13TH SCIENTIFIC MEETING AND EXHIBITION, 7. Mai 2005 (2005-05-07), Seite 1962, XP040594646, * das ganze Dokument * ----- | 5,6 | |
| A | FAUTZ H -P ET AL: "Sliding multislice (SMS): a new technique for minimum FOV usage in axial continuously moving-table acquisitions", MAGNETIC RESONANCE IN MEDICINE WILEY USA, Bd. 55, Nr. 2, Februar 2006 (2006-02), Seiten 363-370, XP002740891, ISSN: 0740-3194 * das ganze Dokument * ----- | 1-12 | RECHERCHIERTE SACHGEBIETE (IPC) G01R |

Der vorliegende Recherchenbericht wurde für alle Patentansprüche erstellt

| Recherchenort | Abschlußdatum der Recherche | Prüfer |
|---|---|---|
| München | 15. Juni 2015 | Skalla, Jörg |

EPO FORM 1503 03.82 (P04C03)

# EP 2 910 966 A1

**ANHANG ZUM EUROPÄISCHEN RECHERCHENBERICHT**
**ÜBER DIE EUROPÄISCHE PATENTANMELDUNG NR.**

EP 15 15 4066

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

15-06-2015

| Im Recherchenbericht angeführtes Patentdokument | Datum der Veröffentlichung | Mitglied(er) der Patentfamilie | | Datum der Veröffentlichung |
|---|---|---|---|---|
| WO 2010001285 A1 | 07-01-2010 | CN | 102084264 A | 01-06-2011 |
| | | EP | 2300842 A1 | 30-03-2011 |
| | | RU | 2011103865 A | 10-08-2012 |
| | | US | 2011112392 A1 | 12-05-2011 |
| | | WO | 2010001285 A1 | 07-01-2010 |
| US 2003011369 A1 | 16-01-2003 | KEINE | | |

Für nähere Einzelheiten zu diesem Anhang : siehe Amtsblatt des Europäischen Patentamts, Nr.12/82

EPO FORM P0461

20